# EUROPEAN PATENT APPLICATION

(11) **EP 3 632 501 A1**
(43) Date of publication of application: **08.04.2020**
(21) Application number: 18809355.3
(22) Date of filing: 28.05.2018
(51) Int. Cl.: A61M 39/10

(54) **FEMALE CONNECTOR**

(30) Priority: 30.05.2017 JP 2017106946
(71) Applicant: JMS Co., Ltd., Hiroshima 730-8652 (JP)
(72) Inventor: UEHARA Yasumasa, Hiroshima-shi, Hiroshima 730-8652 (JP); TAKIMOTO Kazuhiko, Hiroshima-shi, Hiroshima 730-8652 (JP); UKITA Junji, Hiroshima-shi, Hiroshima 730-8652 (JP); KAWACHI Keita, Hiroshima-shi, Hiroshima 730-8652 (JP)
(74) Representative: Dehns
(86) International application number: PCT/JP2018/020302
(87) International publication number: WO 2018/221441

(57) **Abstract**

A female connector (1) includes a female member (10). An inner circumferential face of the female member (1) includes a first region (11), and a second region (12) that is arranged on a tip side of the first region (11). The first region (11) is composed of a female tapered face whose inner diameter increases toward the tip of the female member. The second region (12) is composed of a cylindrical face whose inner diameter is constant along a central axis direction.

## Description

### Technical Field

The present invention relates to a female connector including a female tapered face.

### Background Art

In the field of medicine, flexible tubes or the like are used to form flow channels (alternatively referred to as circuits) through which liquids flow. In order to connect different members, a connecting device constituted by a male connector and a female connector is used. As this sort of connecting device, a connecting device is known in which a male tapered face of a male connector and a female tapered face of a female connector are fitted (taper-fitted) to each other in order to prevent liquid from leaking from a point between the male connector and the female connector (see Patent Document 1, for example). The male tapered face is provided on the outer circumferential face of a tubular male member, and has an outer diameter that decreases toward the tip. The female tapered face is provided on the inner circumferential face of a tubular female member, and has an inner diameter that increases toward the tip. The male tapered face and the female tapered face have the same diameter and the same taper angle. Thus, when the male member is inserted into the female member, the male tapered face and the female tapered face are in surface contact with each other to form a seal therebetween.

For male tapered faces and female tapered faces constituting taper-fitting, there are a plurality of standards with different dimensions (e.g., diameters). In a medical setting, there are cases in which the standards of connecting devices (each constituted by a male connector and a female connector) that are used are different according to a part (e.g., veins, alimentary canals, windpipes, etc.) for which a liquid is administered. The reason for this is to prevent a male connector and a female connector constituting different flow channels from being connected to each other by mistake.

In the standards of male tapered faces and female tapered faces, a nominal value and a tolerance range for the nominal value are defined for dimensions of each constituent element. Manufacturers manufacture male connectors and female connectors within this tolerance range. Thus, even when actually connecting a male connector and a female connector manufactured so as to be fitted to each other, their male tapered face and female tapered face may not always be in surface contact with each other over a wide region. For example, the male tapered face may be fitted to only part of the female tapered face on the tip side (the large-diameter side) or the base end side (the small-diameter side) (this state is referred to as "biased contact" in the present invention).

### Citation List

### Patent Document

Patent Document 1: JP 2012-075495A

### Disclosure of Invention

### Problem to be Solved by the Invention

In a connecting device of a taper-fitting type, the more firmly a male member is inserted into a female member, the more a seal between the male tapered face and the female tapered face improves. At this time, the female member receives a force that increases the diameter, from the male member.

In the female member, the tip portion is weaker than the base end portion. Thus, when the male tapered face is fitted to only part of the female tapered face on the tip side, if the male member is inserted into the female member too firmly, damage such as cracking may occur in the female member.

The female connector including the female member is made of a hard material that is not substantially deformed. As a means for preventing damage to the female member, the entire female connector including the female member may be made of a material (e.g., polypropylene or polythene) with high toughness (i.e., with high tenacity). However, a change in the material of the female connector makes it necessary to change the manufacture method and increase the cost.

A first object of the present invention is to prevent damage to a female member including a female tapered face. A second object of the present invention is to prevent a male connector having a male member that is larger than a male member of a male connector that should have been connected from being connected to a female connector by mistake.

### Means for Solving Problem

The present invention is directed to a female connector including a hollow female member substantially in the shape of a cylinder. The inner circumferential face of the female member includes a first region, and a second region that is arranged on a tip side of the first region.

According to a first female connector of the present invention, the first region is composed of a female tapered face whose inner diameter increases toward the tip of the female member. The second region is composed of a cylindrical face whose inner diameter is constant along a central axis direction.

According to a second female connector of the present invention, the first region is composed of a first female tapered face whose inner diameter increases toward the tip of the female member. The second region is composed of a second female tapered face whose inner diameter increases toward the tip of the female member. The second female tapered face has a taper angle smaller than that of the first female tapered face.

### Effects of the Invention

When a male connector is connected to the female connector of the present invention, the male member of the male connector can be always fitted to the first region of the female member. The first region is positioned in the female member, on the base end side thereof with relatively high strength. Accordingly, it is possible to prevent damage to the female member.

The second region positioned on the tip side of the female member is composed of a cylindrical face or a second female tapered face that has a taper angle smaller than that of the first female tapered face. Thus, the opening diameter on the tip side of the female member is not excessively large. Accordingly, it is possible to prevent misconnection in which a male member that is larger than a male member that should have been connected is connected to a female member by mistake.

### Brief Description of Drawings

[FIG. 1] FIG. 1A is a perspective view of a female connector according to Embodiment 1 of the present invention. FIG. 1B is a cross-sectional view of the female connector according to Embodiment 1 of the present invention. FIG. 1C is a side view of the female connector according to Embodiment 1 of the present invention. FIG. 1D is a front view of the female connector according to Embodiment 1 of the present invention.
[FIG. 2] FIG. 2A is a perspective view of a male connector that is to be connected to the female connector according to Embodiment 1 of the present invention. FIG. 2B is a cross-sectional view of the male connector. FIG. 2C is a side view of the male connector. FIG. 2D is a front view of the male connector.
[FIG. 3] FIG. 3A is a perspective view showing a state in which the male connector is connected to the female connector according to Embodiment 1 of the present invention. FIG. 3B is a cross-sectional view thereof.
[FIG. 4] FIG. 4 is an enlarged cross-sectional view of a male member provided in the male connector shown in FIG. 2A to 2D.
[FIG. 5] FIG. 5 is an enlarged cross-sectional view of a female member provided in a conventional female connector.
[FIG. 6] FIG. 6A is a cross-sectional view showing a state in which the male member shown in FIG. 4 and the conventional female member shown in FIG. 5 are connected to each other through ideal taper-fitting. FIG. 6B is a cross-sectional view showing a state in which the male member shown in FIG. 4 with dimensions not matching the nominal value and the conventional female member shown in FIG. 5 are connected to each other.
[FIG. 7] FIG. 7A is an enlarged cross-sectional view of a female member provided in the female connector according to Embodiment 1 of the present invention. FIG. 7B is a cross-sectional view showing a state in which the male member shown in FIG. 4 and the female member according to Embodiment 1 of the present invention shown in FIG. 7A are connected to each other.
[FIG. 8] FIG. 8A is an enlarged cross-sectional view of a female member provided in a female connector according to a comparative example. FIG. 8B is a cross-sectional view showing a state in which the male member shown in FIG. 4 and the female member according to the comparative example shown in FIG. 8A are connected to each other.
[FIG. 9] FIG. 9 is an enlarged cross-sectional view of a female member provided in a female connector according to Embodiment 2 of the present invention.
[FIG. 10] FIG. 10A is a perspective view of a female connector according to Embodiment 3 of the present invention. FIG. 10B is a cross-sectional view of a female connector according to Embodiment 3 of the present invention.

### Description of the Invention

In the female connector of the present invention, the first region and the second region may be adjacent to each other. This aspect is advantageous in simplifying the configuration of the female connector of the present invention.

The female connector may be capable of being connected to a male connector including a rod-shaped male member. The inner circumferential face of the female member may be configured such that a seal is formed between the first region and the male member when the male connector is connected to the female connector. This aspect is advantageous in preventing damage to the female member because a seal is formed in the first region with relatively high strength. Furthermore, this aspect is advantageous in connecting the female connector and the male connector without leakage of a liquid and a gas.

The inner circumferential face of the female member may be configured such that no seal is formed between the male member and the inner circumferential face excluding the first region when the male connector is connected to the female connector. This aspect is advantageous in preventing damage such as cracking from occurring in the tip portion of the female member with relatively low strength.

An outer circumferential face of the male member may include a male tapered face whose outer diameter decreases toward a tip of the male member. The first region may have a taper angle larger than that of the male tapered face. This aspect is advantageous in improving the seal between the female member and the male member because the region over which the male tapered face is fitted to the first region is relatively narrow.

The female connector of the present invention may further include a contact portion configured to be brought into contact with the male connector, in a direction that is parallel to a longitudinal direction of the male member, substantially at the same time as formation of the seal between the first region and the male member. This aspect is advantageous in further lowering the possibility that damage such as cracking will occur in the female member, because an operator is prevented from firmly inserting the male member into the female member more than is necessary.

An outer circumferential face of the female member may be provided with a protrusion configured to be threaded into a male connector to which the female connector is connected. This aspect is advantageous in firmly connecting the female connector and the male connector and improving the seal between the female member and the male member. According to the present invention, even when the male connector is firmly threaded into the female connector, the possibility that damage will occur in the female member is low.

The female connector may be provided with an indicator that matches an indicator provided in the male connector when the male connector is connected to the female connector. This aspect is advantageous in further lowering the possibility that damage such as cracking will occur in the female member, because an operator is prevented from firmly threading the male connector into the female connector more than is necessary.

Hereinafter, the present invention will be described in detail while showing preferred embodiments thereof. However, it goes without saying that the present invention is not limited to the embodiments below. In the drawings that will be referred to in the following description, only the main members of constituent members of the embodiments of the present invention are shown in a simplified manner for the sake of convenience of description. Accordingly, any member that is not shown in the drawings may be added, and any member shown in the drawings may be changed or omitted, within the scope of the present invention. In the drawings that will be referred to in the description of the embodiments below, members corresponding to those members shown in the drawings that are referred to in the description of any preceding embodiment are denoted by the same reference numerals as the members shown in the drawings of that preceding embodiment. With respect to such members, redundant descriptions are omitted, and the description of the preceding embodiment should be taken into account.

### Embodiment 1

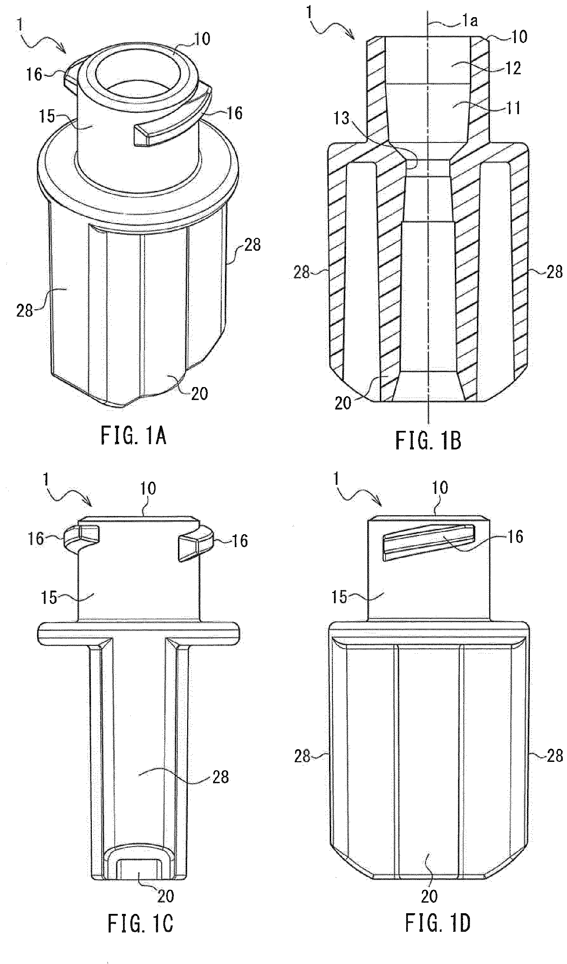

FIG. 1A is a perspective view of a female connector 1 according to Embodiment 1 of the present invention. FIG. 1B is a cross-sectional view of the female connector 1. FIG. 1C is a side view of the female connector 1, and FIG. 1D is a front view of the female connector 1. In FIG. 1B, the dashed dotted line 1a indicates the central axis of the female connector 1. For the sake of convenience of the following description, a direction that is parallel to the central axis 1a is referred to as a "vertical direction". "Up" and "down" are defined based on FIGS. 1A to 1D. Note that "up" and "down" do not mean the actual orientation of the female connector assembly 1 during usage. A direction that is parallel to a plane that is perpendicular to the central axis 1a is referred to as a "horizontal direction". A direction that is orthogonal to the central axis 1a is referred to as a "radial direction" or "diameter direction", and the direction of rotation about the central axis 1a is referred to as a "circumferential direction".

One end of the female connector 1 includes a female member (fittable tube) 10, and the other end thereof includes a connectable tube 20. The female member 10 and the connectable tube 20 are coaxially arranged.

The female member 10 as a whole is hollow and substantially in the shape of a cylinder. The inner circumferential face of the female member 10 includes a first region 11 and a second region 12 with shapes that are different from each other (see FIG. 1B).

The first region 11 is composed of a tapered face (so-called female tapered face) whose inner diameter increases toward the tip (the upper end in FIG. 1B) of the female member 10. Hereinafter, the inner circumferential face of the first region 11 of Embodiment 1 is referred to as a "female tapered face 11". The taper angle of the female tapered face 11 may be freely set, but it may be, for example, 6% tapered.

The second region 12 is on the tip side of, and is adjacent to, the female tapered face 11. The second region 12 is composed of a cylindrical face whose inner diameter is constant along the direction of the central axis 1a, wherein the inner diameter matches the largest inner diameter of the female tapered face 11 (the inner diameter at the tip of the female tapered face 11). Hereinafter, the inner circumferential face of the second region 12 of Embodiment 1 is referred to as a "cylindrical face 12".

The female connector 1 includes a small-diameter portion 13 whose inner diameter is smaller than the smallest inner diameter of the female tapered face 11, on the base end side (the connectable tube 20 side) of the female tapered face 11.

The outer circumferential face of the female member 10 includes a cylindrical face 15 whose outer diameter is constant along the direction of the central axis 1a. The cylindrical face 15 is provided with a radially projecting protrusion 16. The protrusion 16 is a helical protrusion (male thread) helically extending (i.e., in a helical form) about the central axis 1a. In the present invention, the protrusion 16 may extend along the circumferential direction instead of helically extending. Alternatively, the protrusion 16 may be omitted.

Also, the connectable tube 20 as a whole is hollow and substantially in the shape of a cylinder. The connectable tube 20 is connected to the female member 10 via the small-diameter portion 13. A flexible tube (not shown) is to be inserted into the connectable tube 20. The tube is fixed to the inner circumferential face of the connectable tube 20 using an adhesive. The tube constitutes a flow channel through which liquids (e.g., blood, medicinal solutions, nutrients, etc.) flow.

The outer circumferential face of the connectable tube 20 is a substantially cylindrical face. A pair of gripping pieces 28 are provided on the outer circumferential face of the connectable tube 20 so as to radially project in opposite orientations. A cross-section of each gripping piece 28 along the horizontal direction is hollow and substantially in the shape of the latter "U". The pair of gripping pieces 28 and the connectable tube 20 located therebetween constitute a "gripping portion" of the female connector 1. The gripping portion as a whole is a quadrangular prism whose cross-section along the horizontal direction is substantially in the shape of a rectangle. The longitudinal direction of the cross-sectional shape along the horizontal direction is referred to as a "longitudinal direction" of the gripping portion. In Embodiment 1, the long side direction of the substantially rectangular shape, that is, the direction in which the pair of gripping pieces 28 face each other is the longitudinal direction of the gripping portion. The gripping portion makes it possible for an operator to grip the female connector 1 and apply a rotational force thereto with ease.

The configuration of the gripping portion is not limited to Embodiment 1, and may be freely selected. For example, the gripping piece 28 may be a member in the shape of a radially extending thin plate. The gripping portion does not have to be constituted by two gripping pieces 28 and a connectable tube 20 located therebetween. For example, it is also possible that the gripping portion is a hollow quadrangular prism whose cross-section along the horizontal direction is substantially in the shape of a rectangle. In this case, the gripping portion may be arranged so as to surround the connectable tube 20. Also, it is also possible that the gripping pieces 28 are omitted. In this case, the outer circumferential face of the connectable tube 20 may be formed in the shape of, for example, faces of a polygonal prism (faces of a quadrangular prism, faces of a hexagonal prism, etc.), and may be used as the gripping portion.

There is no limitation on the material of the female connector 1, but examples thereof include a material (a hard material) that is hard enough to have mechanical strength (rigidity) that does not substantially allow the female connector 1 to be deformed by an external force. Typically, a material with a hardness of 700 MPa or more is used. Examples thereof include resin materials such as polypropylene (PP), acrylonitrile-butadiene-styrene copolymer (ABS), polycarbonate (PC), polyacetal (POM), polystyrene, polyamide, polythene, and rigid polyvinyl chloride. The female connector 1 can be integrally manufactured such that the entirety is one component through injection molding or the like using the above-described resin materials.

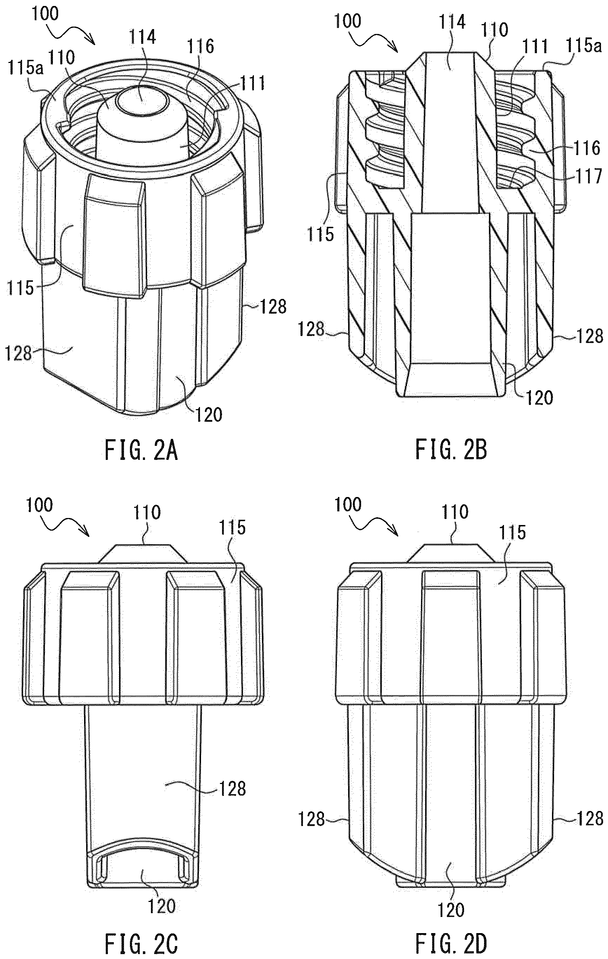

The female connector 1 is connected to a male connector including a male member that is to be fitted to the female member 10. FIGS. 2A to 2D show an example of the male connector. FIG. 2A is a perspective view of a male connector 100, FIG. 2B is a cross-sectional view of the male connector 100, FIG. 2C is a side view of the male connector 100, and FIG. 2D is a front view of the male connector 100. One end of the male connector 100 includes a rod-shaped male member (fittable tube) 110, and the other end thereof includes a connectable tube 120.

The male member 110 as a whole is hollow and substantially in the shape of a cylinder. A flow channel 114 extends through the male member 110 along the longitudinal direction thereof. The outer circumferential face of the male member 110 includes a tapered face (so-called male tapered face) 111 whose outer diameter decreases toward the tip. A cylindrical outer tube 115 surrounds the male member 110. The inner circumferential face that faces the male member 110, of the outer tube 115, is provided with a female thread 116. In the present invention, the outer tube 115 and the female thread 116 may be omitted.

Also, the connectable tube 120 as a whole is hollow and substantially in the shape of a cylinder. The connectable tube 120 is arranged coaxially with the male member 110, and is connected to the flow channel 114. A flexible tube (not shown) is to be inserted into the connectable tube 120. The tube is fixed to the inner circumferential face of the connectable tube 120 using an adhesive.

The outer circumferential face of the connectable tube 120 is a substantially cylindrical face. As in the case of the gripping pieces 28 of the female connector 1, a pair of gripping pieces 128 are provided on the outer circumferential face of the connectable tube 120 so as to radially project in opposite orientations. A cross-section of each gripping piece 128 along the horizontal direction is hollow and substantially in the shape of the latter "U". The pair of gripping pieces 128 and the connectable tube 120 located therebetween constitute a "gripping portion" of the male connector 100. The gripping portion as a whole is a quadrangular prism whose cross-section along the horizontal direction is substantially in the shape of a rectangle. The longitudinal direction of the cross-sectional shape along the horizontal direction is referred to as a "longitudinal direction" of the gripping portion. In Embodiment 1, the long side direction of the substantially rectangular shape, that is, the direction in which the pair of gripping pieces 128 face each other is the longitudinal direction of the gripping portion. The gripping portion makes it possible for an operator to grip the male connector 100 and apply a rotational force thereto with ease. The gripping portion of the male connector 100 is not limited to Embodiment 1, and may be modified or omitted as in the case of the gripping portion of the female connector 1.

There is no limitation on the material of the male connector 100, but examples thereof include a material (a hard material) that is hard enough to have mechanical strength (rigidity) that does not substantially allow the male connector 100 to be deformed by an external force. Specifically, examples thereof may include resin materials that are the same as those given as examples of the material of the female connector 1 described above. The material of the male connector 100 may be the same as or different from that of the female connector 1. The male connector 100 can be integrally manufactured such that the entirety is one component through injection molding or the like using the above-described resin materials.

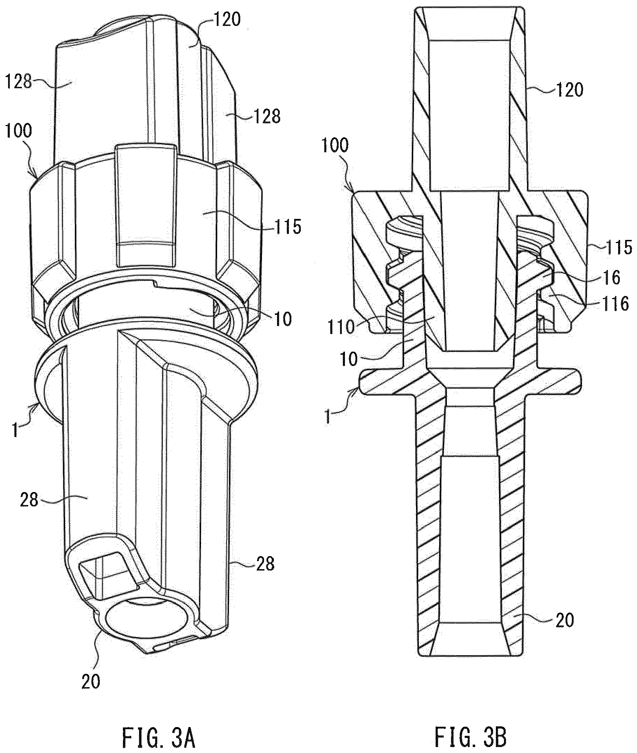

FIG. 3A is a perspective view showing a state in which the male connector 100 is connected to the female connector 1, and FIG. 3B is a cross-sectional view thereof. The female connector 1 and the male connector 100 are connected to each other by inserting the male member 110 into the female member 10 and threading the protrusion 16 and the female thread 116 into each other. The protrusion 16 and the female thread 116 are threaded into each other, thereby firmly connecting the female connector 1 and the male connector 100 and improving the seal between the female member 10 and the male member 110.

As described above, a conventional female member that is taper-fitted to a male member is problematic in that, when the male member is inserted thereinto, damage such as cracking may occur in the female member. The female connector 1 of Embodiment 1 solves this problem by providing the inner circumferential face of the female member 10 with the female tapered face 11 and the cylindrical face 12. Hereinafter, the actions of the female connector 1 of Embodiment 1 will be described in comparison with a conventional female connector.

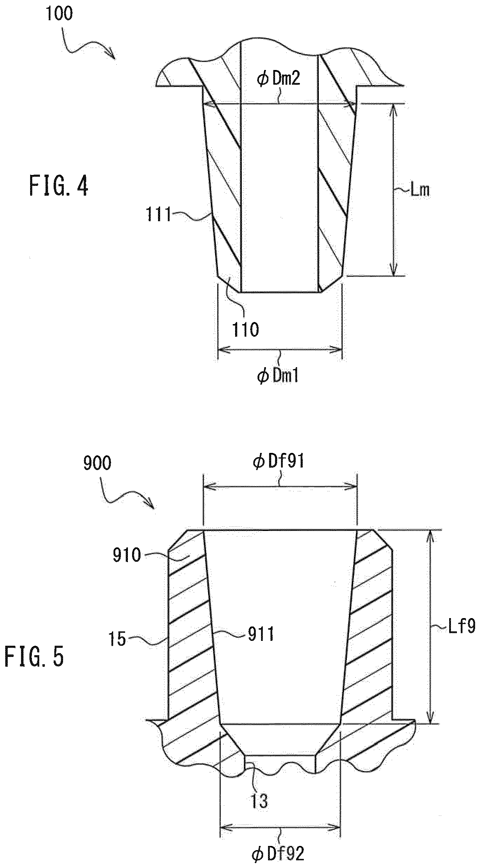

FIG. 4 is an enlarged cross-sectional view of the male member 110 provided in the male connector 100. In FIG. 4, the taper angle of the male tapered face 111 is exaggerated manner than the actual one in order to facilitate understanding. Furthermore, the outer tube 115 and the female thread 116 (see FIG. 2B) are not shown in order to simplify the drawing.

As shown in the drawings, the smallest outer diameter of the male tapered face 111 (the outer diameter at the tip of the male tapered face 111) is φDm1, and the outer diameter of the male tapered face 111 that is away from the point with the smallest outer diameter φDm1 by a length Lm along the central axis toward the base end is φDm2 (φDm2 > φDm1). The diameters φDm1 and φDm2 and the length Lm are nominal values of the standard regarding the male connector 100.

FIG. 5 is an enlarged cross-sectional view of a female member 910 provided in a conventional female connector 900 that is to be fitted to the male connector 100. The inner circumferential face of the conventional female member 910 includes a tapered face (so-called female tapered face) 911 whose inner diameter increases toward the tip (the upper end in FIG. 5) of the female member 910. The tapered face 911 extends to the tip of the female member 910. The cylindrical face 12, provided in the female member 10 of the female connector 1 of Embodiment 1, is not provided in the female member 910. As shown in the drawings, the largest inner diameter of the female tapered face 911 (the inner diameter at the end of the opening of the female member 910) is φDf91 (φDf91 > φDm1), the smallest inner diameter of the female tapered face 911 (the inner diameter at the end on the small-diameter portion 13 side of the female tapered face 911) is φDf92 (φDf92 < φDf91, φDf92 < φDm1), and the length along the central axis of the female tapered face 911 (the distance along the central axis from the point with the largest inner diameter φDf91 to the point with the smallest inner diameter φDf92) is Lf9. The taper angle of the female tapered face 911 matches the taper angle of the male tapered face 111 (see FIG. 4). The diameters φDf91 and φDf92 and the length Lf9 are nominal values of the standard regarding the female connector 900. In FIG. 5, the taper angle of the female tapered face 911 is exaggerated manner than the actual one in order to facilitate understanding. Furthermore, the protrusion 16 provided on the cylindrical face 15 (see FIG. 1A) is not shown in order to simplify the drawing.

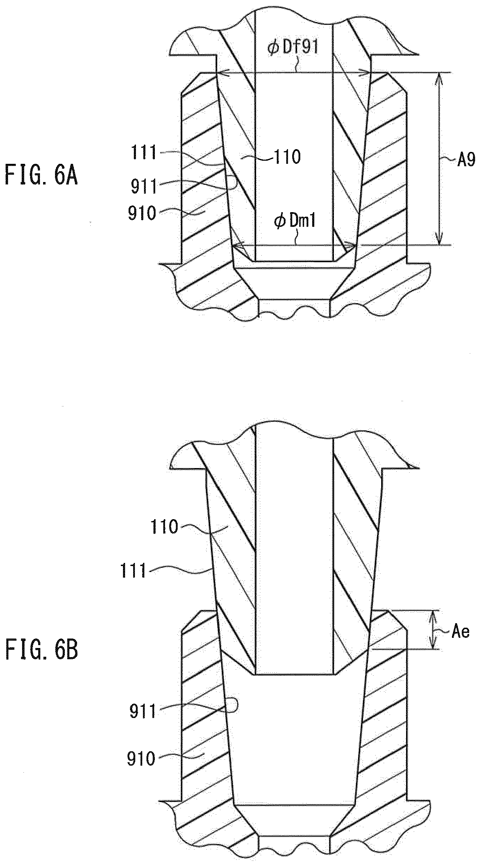

FIG. 6A is a cross-sectional view showing a state in which the male member 110 (see FIG. 4) and the female member 910 (see FIG. 5) are connected to each other through ideal taper-fitting. The male tapered face 111 of the male member 110 and the female tapered face 911 of the female member 910 are in surface contact with each other over a region A9. The region A9 extends from the point at which the male tapered face 111 has the smallest outer diameter φDm1 to the point at which the female tapered face 911 has the largest inner diameter φDf91.

As described above, in the standards of the male member 110 and the female member 910, a nominal value and a tolerance range for the nominal value are defined for dimensions of each constituent element of the male tapered face 111 and dimensions of each constituent element of the female tapered face 911. In a medical setting, the male connector 100 with various dimensions within the tolerance range and the female connector 900 with various dimensions within the tolerance range are connected to each other. Accordingly, the male tapered face 111 and the female tapered face 911 are not always taper-fitted to each other in an ideal manner as shown in FIG. 6A, depending on a combination of the male connector 100 and the female connector 900.

FIG. 6B is a cross-sectional view showing a state in which the male connector 100 with dimensions not matching the nominal value and the female connector 900 are connected to each other. The outer diameter of the male tapered face 111 in FIG. 6B is slightly larger than the nominal value (φDm1, φDm2) of the outer diameter shown in FIG. 4. That is to say, the male tapered face 111 has an outer diameter that is relatively large within the tolerance dimension range. Thus, in FIG. 6B, the male member 110 cannot be inserted into the female member 910 deeply as in FIG. 6A. The male tapered face 111 is fitted to the female tapered face 911 over a relatively narrow region Ae near the tip of the female tapered face 911. In the present invention, a state in which the male tapered face 111 is fitted to only part of the female tapered face 911 on the tip side as in FIG. 6B is referred to as "tip-side biased contact".

In a connecting device of a taper-fitting type in which the male tapered face 111 and the female tapered face 911 are fitted to each other, the more firmly the male member 110 is inserted into the female member 910, the more a seal between the male tapered face 111 and the female tapered face 911 improves. In a tip-side biased contact state as in FIG. 6B, the insertion depth of the male member 110 into the female member 910 is small, and thus an operator is highly likely to firmly push the male member 110 so as to more deeply insert it into the female member 910. If the male member 110 is inserted too firmly into the female member 910 in a tip-side biased contact state, the female member 910 receives a force that increases the diameter thereof, from the male member 110, in the relatively narrow region Ae. The tip portion of the female member 910 is close to the opening of the female member 910, and its wall thickness is thin, and thus, in the female member 910, the tip portion is weaker than the base end portion. Accordingly, damage such as cracking may occur in the female member 910.

The tip-side biased contact state shown in FIG. 6B may occur in a similar manner also in a case in which, for example, the largest inner diameter and the smallest inner diameter of the female tapered face 911 are smaller than their nominal values (φDf91 and φDf92, see FIG. 5) within the tolerance dimension ranges.

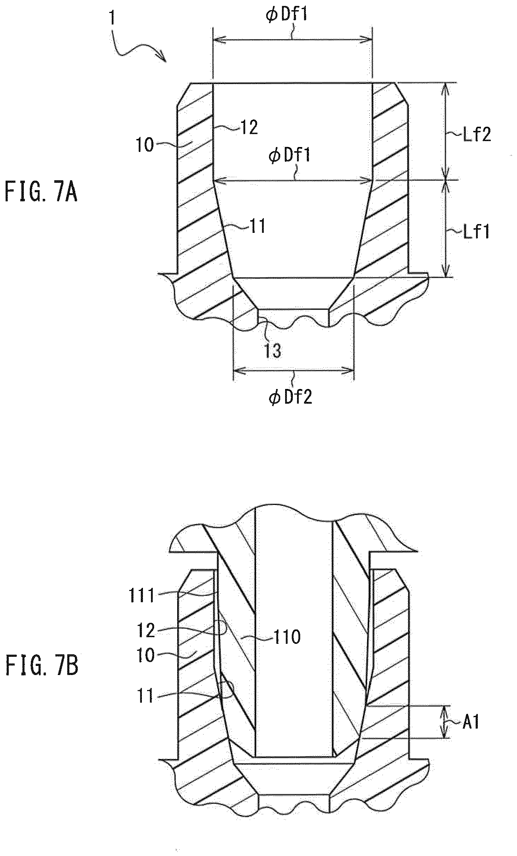

FIG. 7A is an enlarged cross-sectional view of the female member 10 provided in the female connector 1 according to Embodiment 1, designed so as to be fitted to the male connector 100. In FIG. 7A, the taper angle of the female tapered face 11 is exaggerated manner than the actual one in order to facilitate understanding. Furthermore, the protrusion 16 provided on the cylindrical face 15 (see FIG. 1A) is not shown in order to simplify the drawing. As shown in the drawings, the largest inner diameter of the female tapered face 11 (the inner diameter at the end on the cylindrical portion 12 side) is φ)Df1 (φDf1 > φDm1), the smallest inner diameter of the female tapered face 11 (the inner diameter at the end on the small-diameter portion 13 side of the female tapered face 11) is φDf2 (φDf2 < φDf1, φDf2 < φDm1), and the length along the central axis of the female tapered face 11 (the distance along the central axis from the point with the largest inner diameter φDf1 to the point with the smallest inner diameter φDf2) is Lf1 (Lf1 < Lf9). The inner diameter of the cylindrical face 12 is φDf1, and is constant along the direction of the central axis (the central axis 1a in FIG. 1B). The length along the central axis of the cylindrical face 12 is Lf2. In this example, the sum of the length Lf1 of the female tapered face 11 and the length Lf2 of the cylindrical face 12 matches the length Lf9 of the conventional female tapered face 911 (see FIG. 5) (Lf1 + Lf2 = Lf9). The diameters φDf1 and φDf2 and the lengths Lf1 and Lf2 are nominal values of the standard regarding the female connector 1. The diameters φDf1 and φDf2 and the length Lf1 of the female tapered face 11 are set such that the taper angle of the female tapered face 11 is larger than the taper angle of the conventional female tapered face 911 (see FIG. 5) and is larger than the taper angle of the male tapered face 111 (see FIG. 4). Furthermore, the diameter φDf1 and the length Lf2 of the cylindrical face 12 are set such that, when the male member 110 is connected to the female member 10 (see FIG. 7B, which will be described later), the male tapered face 111 does not come into contact with the cylindrical face 12.

FIG. 7B is a cross-sectional view showing a state in which the male member 110 (see FIG. 4) and the female member 10 (see FIG. 7A) are connected to each other. As shown in the drawing, the tip portion (the portion with the smallest outer diameter φDm1 and the vicinity thereof, see FIG. 4) of the male tapered face 111 is fitted to the female tapered face 11 over a region A1. In the region A1, a seal is formed between the female tapered face 11 and the male tapered face 111. The region A1 is narrower than the region A9 in FIG. 6A. Thus, the contact pressure between the female tapered face 11 and the male tapered face 111 in the region A1 increases. This aspect is advantageous in improving the seal between the female tapered face 11 and the male tapered face 111. In the region A1, the male tapered face 111 and/or the female tapered face 11 may be locally deformed.

The male tapered face 111 is fitted to the female tapered face 11. Although not shown, if dimensions of each constituent element of the male tapered face 111 and dimensions of each constituent element of the female tapered face 11 do not match the nominal values within the tolerance dimension ranges, the position of the fitting region A1 of the male tapered face 111 and the female tapered face 11 in the direction of the central axis (the central axis 1a in FIG. 1B) and the dimension of the fitting region A1 in the direction of the central axis change. However, the male tapered face 111 is always fitted to the female tapered face 11, and is not fitted to the cylindrical face 12. That is to say, the region A1 is always positioned on the base end side with relatively high strength, of the female member 10. It is clearly seen from a comparison between FIGS. 7B and 6B that, in Embodiment 1, the female member 10 and the male member 100 are not fitted to each other in a tip-side biased contact state as in FIG. 6B. Accordingly, even when the male member 110 is firmly inserted into the female member 10, the possibility that damage such as cracking will occur in the female member 10 is low.

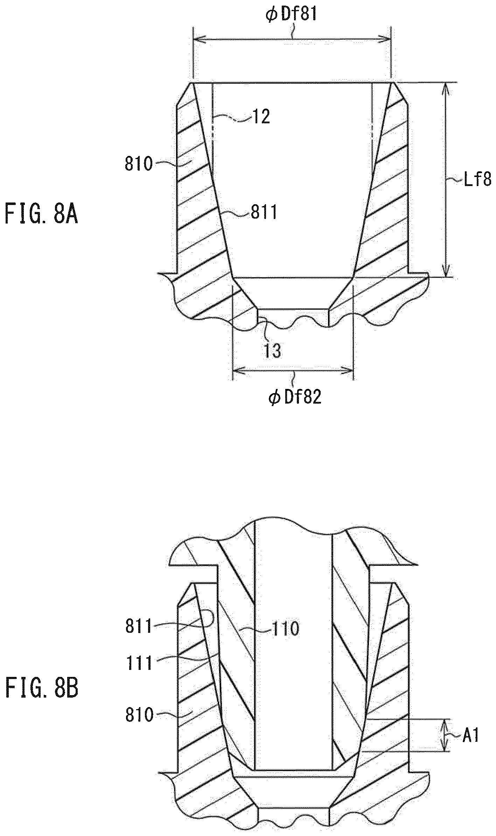

FIG. 8A is an enlarged cross-sectional view of a female member 810 according to a comparative example. The inner circumferential face of the female member 810 is provided with a single female tapered face 811. The taper angle of the female tapered face 811 is the same as the taper angle of the female tapered face 11 of Embodiment 1 shown in FIG. 7A. That is to say, the female tapered face 811 corresponds to a face obtained by extending the female tapered face 11 of the female member 10 of Embodiment 1 (see FIG. 7A) to a region of the cylindrical face 12 (indicated by the dashed double dotted line in FIG. 8A). The largest inner diameter of the female tapered face 811 (the inner diameter at the end of the opening of the female member 810) is φDf81 (φDf81 > φDf1), and the smallest inner diameter of the female tapered face 811 (the inner diameter at the end on the small-diameter portion 13 side of the female tapered face 811) is φDf82 (φDf82 = φDf2, φDf82 < φDm1). The length along the central axis of the female tapered face 811 (the distance along the central axis from the point with the largest inner diameter φDf81 to the point with the smallest inner diameter φDf82) is Lf8 (Lf8 = Lf1 + Lf2).

FIG. 8B is a cross-sectional view showing a state in which the male member 110 (see FIG. 4) and the female member 810 (see FIG. 8A) are connected to each other. As in the case of FIG. 7B, the tip portion of the male the tapered face 911 is fitted to the female tapered face 811 over the region A1. Accordingly, as in the case of the female member 10 of Embodiment 1, even when the male member 110 is firmly inserted into the female member 810, the possibility that damage such as cracking will occur in the female member 810 is low.

The female member 810 has a tip-side opening diameter φDf81 (see FIG. 8A) that is larger than the tip-side opening diameter φDf1 (see FIG. 7A) of the female member 10 of Embodiment 1. Accordingly, it may be possible that another male member (second male member) with an outer diameter that is larger than the male member 110 that should have been connected to the female member 810 can be inserted into the female member 810, and, furthermore, the second male member can be fitted to the female member 810 in a liquid-tight manner. As described above, in a medical setting, there are cases in which the standards (e.g., diameters) of connecting devices (each constituted by a male connector and a female connector) that are used are different according to a part for which a liquid is administered (e.g., veins, alimentary canals, windpipes, etc.). The female member 810 with the large opening diameter φDf81 on the tip side may cause misconnection in which a second male member that is different from the male member 110 that should have been connected is connected to the female member 810.

On the other hand, the inner circumferential face of the female member 10 of Embodiment 1 includes the cylindrical face 12, on the tip side of the female tapered face 11 (see FIG. 7A). Thus, the opening diameter on the tip side of the female member 10 is not excessively large. This aspect is advantageous in lowering the possibility that such misconnection will occur, and provides improved safety compared with the female member 810.

The female connector 1 and the male connector 100 of Embodiment 1 are connected to each other by threading the protrusion 16 into the female thread 116 (see FIG. 3B) until the female tapered face 11 and the male tapered face 111 are fitted to each other over the region A1 (see FIG. 7B). It is preferable that, when the female tapered face 11 and the male tapered face 111 are fitted to each other, the longitudinal direction of the gripping portion of the female connector 1 (i.e., the direction in which the pair of gripping pieces 28 face each other) and the longitudinal direction of the gripping portion of the male connector 1 (i.e., the direction in which the pair of gripping pieces 128 face each other) match each other as shown in FIG. 3A. Accordingly, an operator can see that the female tapered face 11 and the male tapered face 111 are fitted to each other, by rotating the male connector 100 relative to the female connector 1 until the orientation of the gripping portion of the male connector 1 matches the orientation of the gripping portion of the female connector 1. Thus, it is possible to prevent the operator from firmly threading the protrusion 16 into the female thread 116 more than is necessary, which is advantageous in lowering the possibility that damage such as cracking will occur in the female member 10.

As described above, when dimensions of each constituent element of the female tapered face 11 and the male tapered face 111 change within the tolerance ranges, the position of the region A1 in the central axis direction changes, and thus the threading depth of the protrusion 16 into the female thread 116 changes. This change causes dislocation of the gripping portion of the male connector 1 relative to the gripping portion of the female connector 1 in the rotational direction when the female tapered face 11 and the male tapered face 111 are fitted to each other.

Since the taper angle of the female tapered face 11 (see FIG. 7A) is larger than the taper angle of the conventional female tapered face 911 (see FIG. 5), the above-described change in the threading depth of the protrusion 16 into the female thread 116 is smaller in the female connector 1 of Embodiment 1 than in the conventional female connector 900. Thus, even when dimensions of each constituent element of the female tapered face 11 and the male tapered face 111 variously change within the tolerance ranges, the dislocation amount of the male connector 100 relative to the female connector 1 in the rotational direction when the female tapered face 11 and the male tapered face 111 are fitted to each other is small. In other words, there is a high correlation between the fitting state between the female tapered face 11 and the male tapered face 111 and the position of the male connector 100 relative to the female connector 1 in the rotational direction. Accordingly, it is always possible for an operator to properly fit the male tapered face 111 to the female tapered face 11, and to prevent the male connector 100 from being firmly threaded into the female connector 1 more than is necessary, merely by rotating the male connector 100 relative to the female connector 1 such that the orientation of the gripping portion of the female connector 1 and the orientation of the gripping portion of the male connector 100 match each other. Also form this point of view, Embodiment 1 is advantageous in lowering the possibility that damage such as cracking will occur in the female member 10.

### Embodiment 2

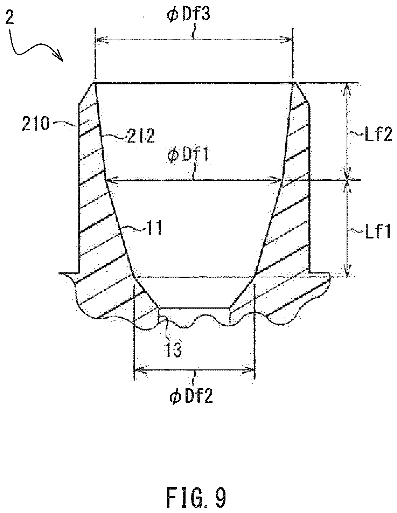

FIG. 9 is an enlarged cross-sectional view of a female member 210 provided in a female connector 2 according to Embodiment 2 of the present invention. According to Embodiment 1, the second region 12 constituting the inner circumferential face of the female member 10 is composed of a cylindrical face. On the other hand, according to Embodiment 2, a second region 212 is composed of a tapered face (so-called female tapered face) whose inner diameter increases toward the tip (the upper end in FIG. 9) of the female member 210. The female tapered face of the first region 11 is referred to as a "first female tapered face 11", and the female tapered face of the second region 212 is referred to as a "second female tapered face 212" so that they are distinguished from each other. The second female tapered face 212 has a taper angle smaller than that of the first female tapered face 11. The smallest inner diameter of the second female tapered face 212 (the inner diameter at the end on the first female tapered face 11 side of the second female tapered face 212) is φDf1, which matches the largest inner diameter of the first female tapered face 11. The largest inner diameter of the second female tapered face 212 (the inner diameter at the end of the opening of the female member 210) is φDf3 (φDf3 > φDm1, φDf3 > φDf1).

When the male member 110 (see FIG. 4) is connected to the female member 210 of Embodiment 2, the male tapered face 111 is always fitted to the first female tapered face 11 and is not fitted to the second female tapered face 212, as in the case of the female member 10 of Embodiment 1 (see FIG. 7B).

The opening diameter φDf3 on the tip side of the female member 210 is smaller than the opening diameter φDf81 on the tip side of the female member 810 according to the comparative example shown in FIG. 8A. Accordingly, as in the case of Embodiment 1, misconnection is unlikely to occur in which a second male member that is different from the male member 110 that should have been connected is connected to the female member 210. Note that, from the viewpoint of more reliably preventing the misconnection, Embodiment 1 is more preferable than Embodiment 2.

According to Embodiment 2, the second region 212 is composed of a female tapered face, and thus, compared with Embodiment 1 in which the second region 12 is composed of a cylindrical face, separation of the female member 210 from a mold (so-called mold releasability) is good in manufacture of a female connector using a resin material through injection molding.

Embodiment 2 is the same as Embodiment 1, except for the above-described aspect. The description of Embodiment 1 applies to Embodiment 2 as well.

### Embodiment 3

According to Embodiment 3, a female connector is provided with a contact portion configured to be brought into contact with the male connector, in a direction that is parallel to a longitudinal direction of the male member, substantially at the same time as formation of the seal between the first region and the male member.

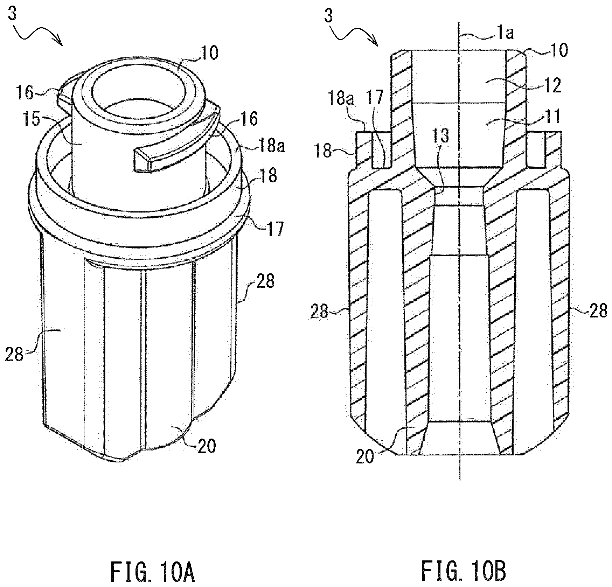

FIG. 10A is a perspective view of a female connector 3 according to Embodiment 3 of the present invention. FIG. 10B is a cross-sectional view of the female connector 3. The female connector 3 includes a ring-like protrusion 18 that is continuous in the circumferential direction, as the above-described contact portion. The protrusion 18 projects upward from (i.e., to the same side as that to which the female member 10 extends) from a disk-like flange 17 projecting radially outward from the base end of the female member 10. The height of the protrusion 18 is constant along the circumferential direction.

As in the case of Embodiment 1, the female connector 3 is connected to the male connector 100. Substantially at the same time as formation of the seal between the female tapered face 11 and the male tapered face 111 (see FIG. 7B), a leading edge 18a of the protrusion 18 is brought into contact with a leading edge 115a of the outer tube 115 of the male connector 100 (see FIGS. 2A and 2B), in the direction of the central axis 1a. When the protrusion 118 is in contact with the outer tube 115, the male member 110 cannot be inserted further deeply into the female member 10. This aspect further lowers the possibility that damage such as cracking will occur in the female member 10.

The contact portion (the protrusion 18) of this embodiment lowers the possibility that an operator will firmly insert the male member into the female member more than is necessary and damage the female member, even if an indicator (e.g., the gripping portion) of the threading depth of a male connector into a female connector is not provided in the female connector and/or the male connector, or even if threading structures which are threaded into each other are not provided in the female connector and the male connector.

The configuration of the contact portion is not limited to the protrusion 18 shown in FIGS. 10A and 10B. For example, the contact portion is not limited to a protrusion that is continuous in the circumferential direction, and also may be a plurality of or one protrusion that is not continuous in the circumferential direction. The contact portion does not have to project toward the male connector. For example, the upper face of the flange 17 may be arranged so as to be brought into contact with the leading edge 115a of the outer tube 115 of the male connector (see FIGS. 2A and 2B) substantially at the same time as formation of the seal between the female tapered face 11 and the male tapered face 111. In this case, the upper face of the flange 17 functions as the contact portion.

In FIGS. 10A and 10B, the contact portion (the protrusion 18) is arranged outside in the radial direction of the female member 10, but the position of the contact portion is not limited to this. For example, the contact portion may be provided at the tip of the female member 10 so as to be brought into contact with an annular flange 117 (see FIG. 2B) that links the base end of the male member 110 and the outer tube 115 of the male connector 100. Alternatively, the contact portion may be provided on the small-diameter portion 13 of the inner circumferential face of the female member 10 or the vicinity thereof so as to be brought into contact with the tip of the male member 110.

Embodiment 3 is the same as Embodiment 1, except for the above-described aspect. The description of Embodiment 1 applies to Embodiment 3 as well. It is also possible to apply the contact portion of this embodiment to the female connector 2 of Embodiment 2.

Embodiments 1 to 3 above are merely examples. The present invention is not limited to Embodiments 1 to 3, and may be changed as appropriate.

For example, in Embodiments 1 to 3 above, the inner circumferential face of the female member includes the first region and the second region that are adjacent to each other, but a third region with a shape different from that of the first region and the second region may be provided therebetween. For example, in Embodiment 1, the third region may be composed of a female tapered face whose inner diameter increases toward the tip. In this case, it is preferable that the taper angle of the female tapered face of the third region is smaller than the taper angle of the female tapered face of the first region. For example, in Embodiment 2, the third region may be composed of a cylindrical face whose inner diameter is constant along the central axis direction.

In Embodiments 1 to 3 above, the male member is configured so as not to be in contact with portions other than the first region, in the inner circumferential face of the female member, when the male member is connected to the female member. However, in the present invention, the male member and portions other than the first region, in the inner circumferential face of the female member may come into contact with each other at a level where no seal is formed therebetween. The reason for this is that contact at a level where no seal is formed does not apply contact pressure that is high enough to damage the female member.

In Embodiments 1 to 3 above, the male member 110 having the male tapered face 111 is connected to the female member. However, male members other than this may be connected to the female member of the female connector of the present invention. For example, a male member having an outer circumferential face that is a cylindrical face whose outer diameter is constant along the central axis direction may be connected to the female member. Also in this case, the male member is fitted to the first region of the female member.

In Embodiments 1 to 3 above, both the female connector and the male connector have gripping portions with similar shapes, and, when the female tapered face 11 and the male tapered face 111 are fitted to each other, the orientation of the gripping portion (in particular, the gripping pieces 28) of the female connector and the orientation of the gripping portion (in particular, the gripping pieces 128) of the male connector match each other. However, the indicator indicating that the female tapered face 11 and the male tapered face 111 have been fitted to each other is not limited to such gripping portions. The indicators may be, for example, marks with any shapes (e.g., a point, a line, a circle, a polygon (a triangle, a rectangle, etc.)) respectively provided at any positions of the female connector and the male connector.

The configuration of the female connector of the present invention, other than the inner circumferential face of the female member, may be freely selected.

The female connector of the present invention is not limited to use in the field of medicine, and can be extensively used in the fields of food and chemistry, as well as various types of machinery, in the case of forming flow channels (circuits) through which fluids flow. The fluids that flow through the female connector is not limited, and may either be a liquid or a gas.

### Industrial Applicability

While there is no particular limitation on the field of use of the present invention, the present invention can be extensively used as a connecting device in which a male member is fitted into a female member and a seal is formed therebetween. Furthermore, the present invention can also be preferably used in the field of medicine as a connecting device for forming a circuit through which various types of liquids flow.

### List of Reference Numerals

- 1,2: Female connector
- 10, 210: Female member
- 11: First region (female tapered face)
- 12: Second region (cylindrical face)
- 16: Protrusion of female member
- 18: Protrusion (contact portion)
- 28: Gripping pieces (indicator)
- 15: Outer circumferential face of female member (cylindrical face)
- 100: Male connector
- 110: Male member
- 111: Male tapered face
- 116: Female thread (threading structure)
- 128: Gripping piece (indicator)
- 212: Second region (female tapered face)

## Claims

1. A female connector comprising a hollow female member substantially in a shape of a cylinder,
wherein an inner circumferential face of the female member includes a first region, and a second region that is arranged on a tip side of the first region,
the first region is composed of a female tapered face whose inner diameter increases toward the tip of the female member, and
the second region is composed of a cylindrical face whose inner diameter is constant along a central axis direction.

2. A female connector comprising a hollow female member substantially in a shape of a cylinder,
wherein an inner circumferential face of the female member includes a first region, and a second region that is arranged on a tip side of the first region,
the first region is composed of a first female tapered face whose inner diameter increases toward the tip of the female member,
the second region is composed of a second female tapered face whose inner diameter increases toward the tip of the female member, and
the second female tapered face has a taper angle smaller than that of the first female tapered face.

3. The female connector according to claim 1 or 2, wherein the first region and the second region are adjacent to each other.

4. The female connector according to any one of claims 1 to 3, wherein the female connector is capable of being connected to a male connector including a rod-shaped male member, and
the inner circumferential face of the female member is configured such that a seal is formed between the first region and the male member when the male connector is connected to the female connector.

5. The female connector according to claim 4, wherein the inner circumferential face of the female member is configured such that no seal is formed between the male member and the inner circumferential face excluding the first region when the male connector is connected to the female connector.

6. The female connector according to claim 4 or 5,
wherein an outer circumferential face of the male member includes a male tapered face whose outer diameter decreases toward a tip of the male member, and
the first region has a taper angle larger than that of the male tapered face.

7. The female connector according to any one of claims 4 to 6, further comprising a contact portion configured to be brought into contact with the male connector, in a direction that is parallel to a longitudinal direction of the male member, substantially at the same time as formation of the seal between the first region and the male member.

8. The female connector according to any one of claims 1 to 7, wherein an outer circumferential face of the female member is provided with a protrusion configured to be threaded into a male connector to which the female connector is connected.

9. The female connector according to claim 8, wherein the female connector is provided with an indicator that matches an indicator provided in the male connector when the male connector is connected to the female connector.
